# EUROPEAN PATENT APPLICATION

(11) **EP 2 976 986 A1**
(43) Date of publication of application: **27.01.2016**
(21) Application number: 14846281.5
(22) Date of filing: 23.04.2014
(51) Int. Cl.: A61B 1/00

(54) **ENDOSCOPE SYSTEM**

(30) Priority: 20.09.2013 JP 2013195740
(71) Applicant: OLYMPUS CORPORATION, Shibuya-ku Tokyo 151-0072 (JP)
(72) Inventor: NAKAJIMA Sho, Tokyo 151-0072 (JP)
(74) Representative: Gunzelmann, Rainer
(86) International application number: PCT/JP2014/061422
(87) International publication number: WO 2015/040882

(57) **Abstract**

An endoscope system 1 includes an insertion portion 5, a detection fiber 13 that transmits light received by a distal end face 13a, a beam splitter 28 that can divide the light transmitted by the detection fiber 13 into a first optical path and a second optical path, and guide the light, a detection fiber 14 that transmits light received by a distal end face 14a, a beam splitter 29 that can reflect the light transmitted by the detection fiber 14 into a third optical path oriented in the same direction as the first optical path and guide the light, and make light from an auxiliary light source 31 provided on a proximal end side of the detection fiber 14 incident on a proximal end portion of the detection fiber 14, detectors 47a to 47c that can detect light intensities of light from the first optical path and the third optical path, and an auxiliary image pickup device 30 that can receive light from the second optical path.

## Description

### Technical Field

The present invention relates to an endoscope system that scans laser light.

### Background Art

As is publicly known, there are electronic endoscopes that photoelectrically convert an object image using an image pickup apparatus having a solid image pickup device such as CCD, CMOS and display the acquired image on a monitor. In recent years, scanning type endoscopes are available as apparatuses that display an object image without using techniques of such a solid image pickup device. This scanning type endoscope causes a distal end of an illumination fiber that guides illuminating light from a light source to scan, receives return light from the object using a detection fiber arranged around the illumination fiber, and images the object image using chronologically detected light intensity signals.

The scanning type endoscope provides a drive section such as an actuator that displaces the illumination fiber to scan the illuminating light in a distal end portion of an insertion portion, radiates illuminating light such as laser light from an illumination lens at the distal end of the insertion portion, and thereby illuminates the object.

For example, Japanese Patent Application Laid-Open Publication No. 2011-115252 discloses a scanning type medical probe which is provided with a single mode fiber for transmitting illuminating light into a body cavity, emits the illuminating light while keeping a distal end of the single mode fiber resonating, and thereby scans the object in a predetermined scanning pattern.

However, when the illumination fiber is broken or the actuator that displaces the illumination fiber to scan the illuminating light is broken, such a scanning type medical probe can no longer scan the illuminating light. An operator cannot observe the object image and needs to pay close attention to safely removing the insertion portion.

In order to solve such a problem, for example, a method is available which additionally provides an auxiliary light source and an auxiliary image pickup device or the like as a separate system, and observes an object image in the event of a malfunction using these auxiliary light source and auxiliary image pickup device.

However, when the auxiliary light source and the auxiliary image pickup device are additionally provided, an illumination fiber and a detection fiber need to be newly added to the insertion portion, which causes an outside diameter of the insertion portion to increase, preventing the insertion portion from being inserted into a narrow duct.

It is therefore an object of the present invention to provide an endoscope system capable of preventing the outside diameter of the insertion portion from increasing and safely removing the insertion portion even when scanning of laser light is stopped.

### Disclosure of Invention

### Means for Solving the Problem

An endoscope system according to an aspect of the present invention includes an elongated insertion portion that is inserted into a portion to be observed, a first light-receiving section that is provided at a distal end portion of the insertion portion and can receive light from an object to be observed, a first light transmitting section that is inserted into the insertion portion and transmits the light received by the first light-receiving section, a first light guide section that divides the light transmitted by the first light transmitting section into a first optical path and a second optical path and can guide the light, a second light-receiving section that is provided at the distal end portion and can receive the light from the object to be observed, a second light transmitting section that is inserted into the insertion portion and transmits the light received by the second light-receiving section, a second light guide section that reflects the light transmitted by the second light transmitting section into a third optical path oriented in the same direction as the first optical path, can guide the light and can make light from a light source provided on a proximal end side of the second light transmitting section incident on a proximal end portion of the second light transmitting section, a photodetector that can detect light intensities of the light from the first optical path and the third optical path, and an image pickup device that can receive the light from the second optical path.

### Brief Description of the Drawings

Fig. 1 is a diagram for describing an overall configuration of an endoscope system according to a first embodiment;
Fig. 2 is a diagram for describing a distal end configuration of an endoscope according to the first embodiment;
Fig. 3 is a flowchart for describing operation of the endoscope system 1; and
Fig. 4 is a diagram for describing a distal end configuration of an endoscope according to a second embodiment.

### Best Mode for Carrying Out the Invention

Hereinafter, embodiments of the present invention will be described with reference to the accompanying drawings.

### (First Embodiment)

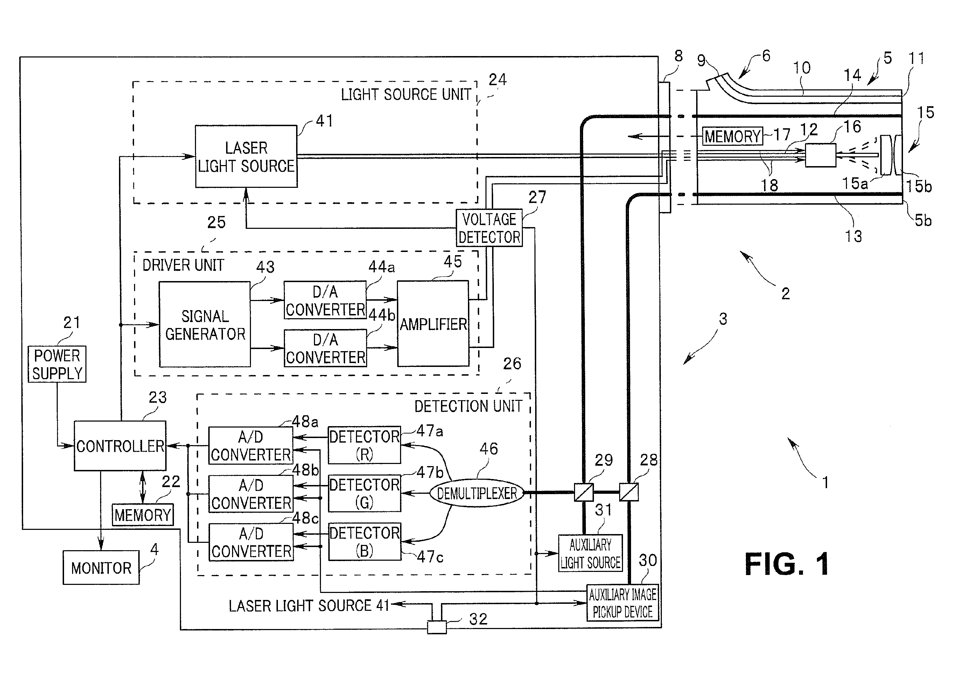

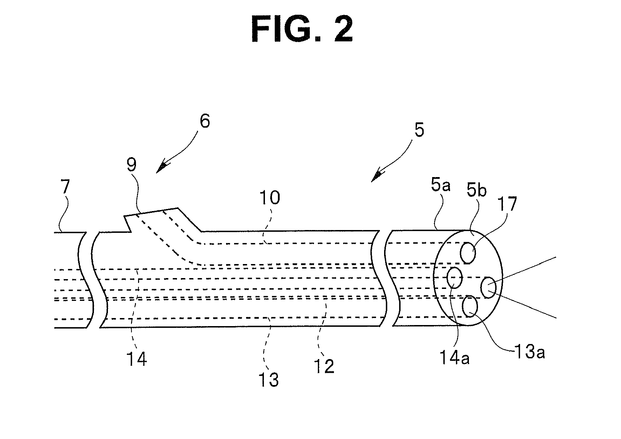

First, an overall configuration of an endoscope system according to a first embodiment will be described using Fig. 1 and Fig. 2.

Fig. 1 is a diagram for describing an overall configuration of the endoscope system according to the first embodiment and Fig. 2 is a diagram for describing a distal end configuration of an endoscope according to the first embodiment.

As shown in Fig. 1, an endoscope system 1 is constructed by including a scanning type endoscope 2 that radiates laser light (illuminating light) onto an object to be observed while scanning the laser light and obtains return light from the object to be observed, a main body apparatus 3 connected to this endoscope 2 and a monitor 4 that displays an image of the object to be observed obtained in the main body apparatus 3.

The endoscope 2 is principally constructed of a tube body provided with predetermined flexibility, and includes an elongated insertion portion 5 which is inserted into the object to be observed, an operation portion 6 which is connected on a proximal end side of the insertion portion 5, a flexible tube portion 7 connected on a proximal end side of the operation portion 6 (see Fig. 2), and a connector 8 connected on a proximal end side of the flexible tube portion 7. This connector 8 is detachably attached to the main body apparatus 3.

The operation portion 6 is provided with a treatment instrument insertion port 9 to insert a treatment instrument such as forceps into the insertion portion 5. A treatment instrument insertion channel 10 is inserted into the insertion portion 5 as a duct which communicates with the treatment instrument insertion port 9 and through which the treatment instrument can be inserted. Furthermore, an opening portion 11 is provided on a distal end face 5b of a distal end portion 5a of the insertion portion 5, which includes an opening communicating with the treatment instrument insertion channel 10.

As shown in Fig. 2, an optical fiber 12 is inserted into the insertion portion 5, which guides light from a laser light source 41, which will be described later, of the main body apparatus 3 and radiates laser light onto the object to be observed. A detection fiber 13 is also inserted into the insertion portion 5, which receives return light from the object to be observed by a distal end face 13a as a first light-receiving section. The detection fiber 13 as a first light transmitting section guides the return light received by the distal end face 13a to a beam splitter 28, which will be described later, of the main body portion 3.

Furthermore, a detection fiber 14 is inserted into the insertion portion 5, which receives the return light from the object to be observed by a distal end face 14a as a second light-receiving section. The detection fiber 14 as a second light transmitting section guides the return light received by the distal end face 14a to a beam splitter 29, which will be described later, of the main body portion 3.

Returning to Fig. 1, a distal end optical system 15 made up of illumination lenses 15a and 15b is provided on the distal end face 5b of the insertion portion 5. In the distal end optical system 15, an illumination lens 15a which is a plano-convex lens and an illumination lens 15b which is a convex-plano lens are arranged in that order from the laser light source 41 side, which will be described later. Note that although the distal end optical system 15 is constructed of the two illumination lenses 15a and 15b, the distal end optical system 15 is not limited to this, but may be constructed of one or three or more illumination lenses.

An actuator 16 is provided on the distal end side of the optical fiber 12 that guides light from the laser light source 41, which will be described later, and radiates the laser light onto the object to be observed, the actuator 16 functioning as a drive section that causes the distal end of the optical fiber 12 to scan in a desired direction based on a drive signal from a driver unit 25, which will be described later. In this way, the actuator 16 constitutes an illuminating light radiation section that can scan and emit laser light.

Although not illustrated, for example, a ferrule shaped like a quadrangular prism is arranged between the optical fiber 12 and the actuator 16, and the actuator 16 is arranged on each side face of the ferrule.

This ferrule is a member used in the optical communication field and zirconia (ceramic), nickel or the like is used as its material, which allows a central hole to be easily machined with high accuracy (e.g., ±1 µm) with respect to an outside diameter of the optical fiber 12 (e.g., 125 µm). A through hole based on the diameter of the optical fiber 12 is provided at substantially the center of the ferrule and the optical fiber 12 is fixed thereto using an adhesive or the like.

Furthermore, a memory 17 storing various kinds of information relating to the endoscope 2 is provided in the operation portion 6. When the endoscope 2 is connected to the main body apparatus 3, the memory 17 is connected to a controller 23, which will be described later, via a signal line which is not illustrated, and the various kinds of information relating to the endoscope 2 is read by the controller 23.

A plurality of lead wires 18 made of, for example, linear metal are vapor-deposited onto the optical fiber 12 from the connector 8 to the actuator 16. The actuator 16 is provided at a distal end of the plurality of lead wires 18. When the connector 8 is attached to the main body apparatus 3, the plurality of lead wires 18 are connected to an amplifier 45 via a voltage detector 27, which will be described later, of the main body apparatus 3. The plurality of lead wires 18 supply, to the actuator 16, drive signals (drive voltages) outputted from the amplifier 45 to drive the actuator 16.

The main body apparatus 3 is constructed by including a power supply 21, a memory 22, the controller 23, a light source unit 24, a driver unit 25, a detection unit 26, the voltage detector 27, the beam splitter 28, the beam splitter 29, an auxiliary image pickup device 30, an auxiliary light source 31, and an auxiliary observation manual changeover switch 32.

The light source unit 24 is constructed by including the laser light source 41. The driver unit 25 is constructed by including a signal generator 43, digital/analog (hereinafter referred to as "D/A") converters 44a and 44b, and the amplifier 45.

The detection unit 26 is constructed by including a demultiplexer 46, detectors 47a to 47c, and analog/digital (hereinafter referred to as "A/D") converters 48a to 48c.

The power supply 21 controls a power supply to the controller 23 according to operation of a power supply switch which is not illustrated or the like. The memory 22 stores a control program or the like to control the entire main body apparatus 3.

Upon receiving a power supply from the power supply 21, the controller 23 reads the control program from the memory 22, controls the light source unit 24 and the driver unit 25, analyzes light intensity of the return light from the object detected by the detection unit 26, and performs control to cause the monitor 4 to display the object image obtained.

The laser light source 41 of the light source unit 24 emits laser light (illuminating light) of a predetermined wavelength band to the optical fiber 12 based on the control of the controller 23. This optical fiber 12 emits the laser light (illuminating light) from the laser light source 41 to the object to be observed via the illumination lenses 15a and 15b.

The signal generator 43 of the driver unit 25 outputs a drive signal for causing the distal end of the optical fiber 12 to scan (scanning drive) in a desired direction, for example, in a spiral shape based on the control of the controller 23. More specifically, the signal generator 43 outputs, to the D/A converter 44a, a drive signal for causing the distal end of the optical fiber 12 to drive in a horizontal direction (X-axis direction) with respect to an insertion axis of the insertion portion 5, and outputs, to the D/A converter 44b, a drive signal for causing the distal end of the optical fiber 12 to drive in a vertical direction (Y-axis direction) with respect to the insertion axis of the insertion portion 5.

The D/A converters 44a and 44b each convert an inputted drive signal from a digital signal to an analog signal and output the analog signal to the amplifier 45. The amplifier 45 amplifies the inputted drive signal and outputs the drive signal to the actuator 16 via the voltage detector 27. Note that the voltage detector 27 will be described later. The drive signal outputted from the amplifier 45 is supplied to the actuator 16 via the plurality of lead wires 18 which are vapor-deposited to the optical fiber 12.

The actuator 16 causes the distal end of the optical fiber 12 to oscillate and scan in a spiral shape based on the drive signal from the amplifier 45. More specifically, the actuator 16 vibrates together with the optical fiber 12 and drives the optical fiber 12 in a diameter direction so that the illuminating light emitted from the optical fiber 12 scans in a region to be observed. In this way, light emitted from the laser light source 41 of the light source unit 24 to the optical fiber 12 is sequentially radiated onto the object to be observed in a spiral shape.

The detection fiber 13 receives the return light reflected by the surface region of the object to be observed by the distal end face 13a and guides the received return light to the beam splitter 28. Similarly, the detection fiber 14 receives the return light reflected by the surface region of the object to be observed by the distal end face 14a and guides the received return light to the beam splitter 29.

The beam splitter 28 always divides the return light guided from the detection fiber 13 at a predetermined proportion during both normal observation and auxiliary observation and guides the split beams to the beam splitter 29 and the auxiliary image pickup device 30. The normal observation corresponds to a state in which observation is performed by scanning the laser light from the laser light source 41 and the auxiliary observation corresponds to a state in which observation is performed by driving the auxiliary image pickup device 30 and the auxiliary light source 31 when, for example, the actuator 16 or the like malfunctions and cannot cause laser light to scan. The predetermined proportion is optional and the beam splitter 28 divides the return light guided from the detection fiber 13 into 50% portions, for example, and guides the portions to the beam splitter 29 and the auxiliary image pickup device 30.

Thus, the beam splitter 28 constitutes a first light guide section that can divide the return light transmitted by the detection fiber 13 into a first optical path guided to the beam splitter 29 and a second optical path guided to the auxiliary image pickup device 30.

Note that the beam splitter 28 may be a reflector, for example. During normal operation, all the return light guided from the detection fiber 13 is guided (reflected) to the beam splitter 29 through a reflector, and when a malfunction occurs, the reflector is deviated from the optical axis using the actuator so that all the return light guided from the detection fiber 13 is guided to the auxiliary image pickup device 30.

During normal observation, the beam splitter 29 guides (reflects) all the return light guided from the detection fiber 14 to the demultiplexer 46 of the detection unit 26. During normal observation, the beam splitter 29 allows to pass, the return light guided from the detection fiber 13 which has been divided by the beam splitter 28 at the predetermined proportion and guides the return light to the demultiplexer 46.

On the other hand, during auxiliary observation, the beam splitter 29 allows the illuminating light emitted from the auxiliary light source 31 to pass as is and guides the illuminating light to the detection fiber 14. The illuminating light guided to the detection fiber 14 is emitted from the distal end face 14a and radiated into the object to be observed.

Thus, the beam splitter 29 constitutes a second light guide section that can reflect the return light transmitted through the detection fiber 14 into a third optical path oriented in the same direction as the first optical path and guide the return light, and can also make the illuminating light from the auxiliary light source 31 provided on the proximal end side of the detection fiber 14 incident on the proximal end portion of the detection fiber 14.

During auxiliary observation, the auxiliary image pickup device 30 is driven based on an auxiliary observation changeover signal, which will be described later, picks up an image of the return light guided from the detection fiber 13 divided by the beam splitter 28 at the predetermined proportion and outputs an image pickup signal to the A/D converters 48a to 48c. Thus, the auxiliary image pickup device 30 constitutes an image pickup device that can receive the return light guided from the detection fiber 13 (light from the aforementioned second optical path). Note that the type of the image pickup device used for the auxiliary image pickup device 30 is not particularly limited, and can be a CCD or CMOS, for example.

Furthermore, the auxiliary light source 31 is driven during auxiliary observation based on an auxiliary observation changeover signal, which will be described later, and emits illuminating light to the detection fiber 14 via the beam splitter 29. Note that the type of the light source used for the auxiliary light source 31 is not particularly limited, but can be a xenon lamp, halogen lamp or LED, for example.

The auxiliary image pickup device 30 and the auxiliary light source 31 are respectively provided with an image pickup device and a light source that provide a minimum degree of image quality and brightness to prevent manipulation from being continued in a malfunctioning condition in which it is not possible to scan laser light.

More specifically, a light source that emits illuminating light of brightness equal to or lower than the brightness necessary to illuminate areas surrounding a fine duct up to several millimeters ahead of the distal end face 5b of the insertion portion 5 is used for the auxiliary light source 31. Furthermore, an illumination optical system is used which is optically designed so as to intentionally reduce the brightness in the vicinity of the center used for a front view or avoid illumination and radiate only peripheral light that illuminates areas surrounding the duct necessary to remove the insertion portion 5 from the duct. Also for the auxiliary image pickup device 30, an image pickup device is used, which is optically designed to perform electrical control so as to intentionally reduce the sensitivity in the vicinity of the center or avoid image formation or optically designed to perform electrical control so as to be sensitive only to areas surrounding the duct necessary to remove the insertion portion 5 from the duct or form an image.

The voltage detector 27 detects a drive voltage from the amplifier 45 and also detects a drive voltage that is actually applied to the actuator 16. When a malfunction that prevents scanning of laser light such as breakage of the optical fiber 12, damage to the actuator 16 or wire breakage of the lead wires 18 occurs, a change occurs in the drive voltage actually applied to the actuator 16.

For this reason, when the drive voltage from the amplifier 45 is the same as the drive voltage actually applied to the actuator 16, the voltage detector 27 determines that no malfunction has occurred in the optical fiber 12, the actuator 16 or the lead wires 18. On the other hand, when the drive voltage from the amplifier 45 is not the same as the drive voltage actually applied to the actuator 16, the voltage detector 27 determines that a malfunction has occurred in the optical fiber 12, the actuator 16 or the lead wires 18.

Upon determining that a malfunction has occurred, the voltage detector 27 outputs an auxiliary observation changeover signal for switching the operation from normal observation to auxiliary observation to the laser light source 41, the auxiliary image pickup device 30 and the auxiliary light source 31. More specifically, the voltage detector 27 outputs a signal for causing the laser light source 41 to stop emitting laser light, outputs a signal for causing the auxiliary image pickup device 30 to start image pickup and outputs a signal for causing the auxiliary light source 31 to start emitting illuminating light. In this way, the auxiliary light source 31 emits illuminating light and the auxiliary image pickup device 30 picks up an image of return light and performs auxiliary observation.

In this way, the voltage detector 27 constitutes a detection section that detects if there is any abnormality in scanning of laser light and can drive the auxiliary image pickup device 30 and the auxiliary light source 31.

Note that warning means may also be provided for warning, when the voltage detector 27 detects a malfunction (abnormality in the drive voltage), of the malfunction. This warning means is intended to cause the operator to immediately recognize the abnormality by sounding a warning beep from a speaker or displaying a message like "Please remove the endoscope" on the monitor 4.

The auxiliary observation manual changeover switch 32 is a switch for manually switching the operation from normal observation to auxiliary observation. That is, when the voltage detector 27 cannot switch the operation from normal observation to auxiliary observation despite the fact that no observation image is displayed on the monitor 4, the auxiliary observation manual changeover switch 32 is a switch for manually switching the operation from normal observation to auxiliary observation.

Even when no malfunction such as breakage of the optical fiber 12, damage to the actuator 16 or wire breakage of the lead wires 18, has occurred, if, for example, a malfunction occurs in the laser light source 41, no laser light is emitted from the optical fiber 12 to the object to be observed, and therefore no observation image is displayed on the monitor 4. However, since no malfunction has occurred in the optical fiber 12, the actuator 16 or the lead wires 18, the voltage detector 27 cannot detect any abnormality in the drive voltage. For this reason, the voltage detector 27 does not output any auxiliary observation changeover signal to the laser light source 41, the auxiliary image pickup device 30 and the auxiliary light source 31, and the operation is not switched from normal observation to auxiliary observation.

Furthermore, even when a malfunction such as breakage of the optical fiber 12, damage to the actuator 16 or wire breakage of the lead wires 18, has occurred, if the voltage detector 27 malfunctions, no auxiliary observation changeover signal is outputted from the voltage detector 27 to the laser light source 41, the auxiliary image pickup device 30 and the auxiliary light source 31, and the operation is not switched from normal observation to auxiliary observation. In such a case, the operator presses the auxiliary observation manual changeover switch 32, and can thereby switch the operation from normal observation to auxiliary observation.

When the operator presses the auxiliary observation manual changeover switch 32, the auxiliary observation manual changeover switch 32 outputs a signal for causing the laser light source 41 to stop emitting laser light, outputs a signal for causing the auxiliary image pickup device 30 to start image pickup and outputs a signal for causing the auxiliary light source 31 to start emitting illuminating light. This allows the operation to be switched from normal observation to auxiliary observation.

Thus, the auxiliary observation manual changeover switch 32 constitutes a starting section that drives the auxiliary image pickup device 30 and the auxiliary light source 31 only when there is an abnormality in scanning of laser light.

Note that, in order to prevent wrong switching during normal observation, pressing of the auxiliary observation manual changeover switch 32 may be validated, for example, only when a signal supply to the monitor 4 is stopped. Furthermore, when a signal supply to the monitor 4 is stopped, operation buttons other than the auxiliary observation manual changeover switch 32 may be invalidated. This can restrain the auxiliary observation manual changeover switch 32 from being used at any time other than during auxiliary observation during which the insertion portion 5 of the endoscope 2 is safely removed.

The demultiplexer 46 is, for example, a dichroic mirror and demultiplexes return light in a predetermined wavelength band. More specifically, the demultiplexer 46 demultiplexes the return light guided from the beam splitters 28 and 29 into return light in R, G and B wavelength bands and outputs the return light to the detectors 47a, 47b and 47c respectively.

The detectors 47a, 47b and 47c detect light intensities of the return light in R, G and B wavelength bands respectively. Thus, the detectors 47a, 47b and 47c constitute a photodetector that can detect light intensity of return light guided from the beam splitters 28 and 29 (light in the aforementioned first and second optical paths). The signals of the light intensities detected by the detectors 47a, 47b and 47c are outputted to the A/D converters 48a, 48b and 48c respectively.

The A/D converters 48a to 48c respectively convert the signals of the light intensities outputted from the detectors 47a to 47c from analog signals to digital signals during normal observation and output the digital signals to the controller 23. On the other hand, the A/D converters 48a to 48c convert the image pickup signal outputted from the auxiliary image pickup device 30 from an analog signal to a digital signal during auxiliary observation and output the digital signal to the controller 23.

The controller 23 applies predetermined image processing on the digital signals from the A/D converters 28a to 28c, generates an object image and displays the object image on the monitor 4. The digital signals from the A/D converters 28a to 28c are signals outputted from the detectors 47a to 47c during normal observation, and signals outputted from the auxiliary image pickup device 30 during auxiliary observation. Thus, the monitor 4 constitutes a display apparatus that can display either a first output according to the information acquired by the detectors 47a to 47 or a second output according to the information acquired by the auxiliary image pickup device 30.

Note that the controller 23 may make the color balance of the observation image during auxiliary observation different from the color balance of the observation image during normal observation. More specifically, an observation image whose prevailing color is a complementary color exactly the opposite to the intravital color scheme during normal observation is used during auxiliary observation, or an observation image whose prevailing color is blue is used with respect to red which is the intravital base color. This allows the operator to immediately recognize that the operation has been switched from normal observation to auxiliary observation and makes color information which is important to distinguish each tissue of a living body different from a normal color scheme, and can thereby prevent the color information from being used for purposes other than auxiliary observation during which the insertion portion 5 of the endoscope 2 can be safely removed.

Here, operation of the endoscope system 1 configured as described above will be described.

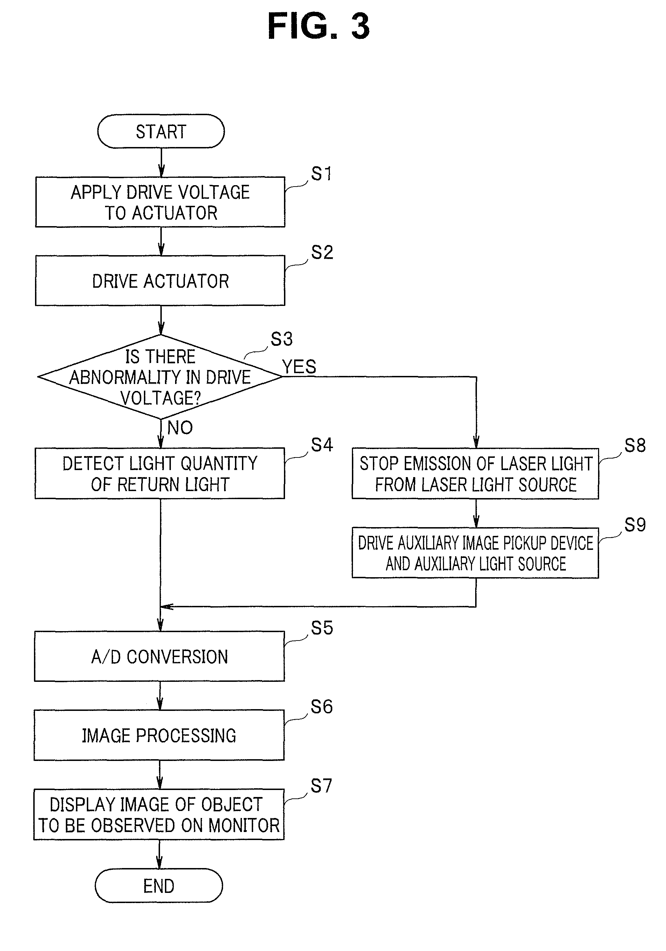

Fig. 3 is a flowchart for describing operation of the endoscope system 1.

First, a drive voltage is applied to the actuator 16 from the amplifier 45 of the driver unit 25 (step S1) to drive the actuator 16 (step S2). Next, the voltage detector 27 detects the drive voltage outputted from the amplifier 45 and the drive voltage actually applied to the actuator 16, and determines whether or not there is any abnormality in the drive voltage (step S3). When the drive voltage outputted from the amplifier 45 is the same as the drive voltage actually applied to the actuator 16, the voltage detector 27 determines that there is no abnormality in the drive voltage, whereas when the drive voltage outputted from the amplifier 45 is not the same as the drive voltage actually applied to the actuator 16, the voltage detector 27 determines that there is an abnormality in the drive voltage.

When it is determined in step S3 that there is no abnormality in the drive voltage, that is, NO, the detectors 47a to 47c detect a light quantity of the return light from the object to be observed (step S4). The return light from the object to be observed is a merge of the return light guided by the detection fiber 13 and divided by the beam splitter 28 at a predetermined proportion and the return light guided by the detection fiber 14 and totally reflected by the beam splitter 29.

The light quantity of the return light is A/D-converted by the A/D converters 48a to 48c (step S5), and subjected to image processing by the controller 23 (step S6). Lastly, the image of the object to be observed subjected to image processing by the controller 23 is displayed on the monitor 4 (step S7), and the process ends.

On the other hand, when it is determined in step S3 that there is an abnormality in the drive voltage, that is, YES, emission of laser light from the laser light source 41 is stopped (step S8), and the auxiliary image pickup device 30 and the auxiliary light source 31 are driven (step S9). Processes in steps S8 and S9 are executed by the voltage detector 27 (or auxiliary observation manual changeover switch 32) outputting an auxiliary observation changeover signal to the laser light source 41, the auxiliary image pickup device 30 and the auxiliary light source 31.

Thus, when emission of laser light from the laser light source 41 is stopped, the auxiliary light source 31 is driven and illuminating light is emitted. The illuminating light from the auxiliary light source 31 passes through the beam splitter 29, is guided by the detection fiber 14 and radiated onto the object to be observed. The return light from the object to be observed is guided from the detection fiber 13 to the beam splitter 28, divided at a predetermined proportion and then guided to the auxiliary image pickup device 30.

Since the auxiliary image pickup device 30 is driven according to an inputted auxiliary observation changeover signal, the auxiliary image pickup device 30 picks up an image of the guided return light and outputs the image pickup signal to the A/D converters 48a to 48c. After that, processes similar to those in steps S5 to S7 are executed and the image of the object to be observed picked up by the auxiliary image pickup device 30 is displayed on the monitor 4.

As described above, in the endoscope system 1, the voltage detector 27 detects a malfunction that prevents laser light from being scanned, such as breakage of the optical fiber 12, damage to the actuator 16 or wire breakage of the lead wires 18, and switches the operation from normal observation to auxiliary observation. In auxiliary observation, one detection fiber 14 is used as an illumination fiber that guides illuminating light from the auxiliary light source 31 and the auxiliary image pickup device 30 picks up an image of the return light from the other detection fiber 13.

As a result, the endoscope system 1 need not newly provide an illumination fiber used during auxiliary observation in the insertion portion 5, and so never increases the diameter of the insertion portion 5 of the endoscope 2. Furthermore, the endoscope system 1 is provided with the auxiliary light source 31 that emits minimum necessary illuminating light to remove the insertion portion 5 from the object to be observed even in the event of a malfunction that prevents laser light from the laser light source 41 from being scanned and the auxiliary image pickup device 30 that picks up an image of the return light from the object to be observed. Thus, even in the event of a malfunction that prevents laser light from being scanned, the image of the object to be observed picked up by the auxiliary image pickup device 30 can be displayed on the monitor 4, and it is thereby possible to safely remove the insertion portion 5 from the object to be observed.

Thus, according to the endoscope system of the present embodiment, it is possible to prevent the outside diameter of the insertion portion from increasing and safely remove the insertion portion even when scanning of laser light is stopped.

### (Second Embodiment)

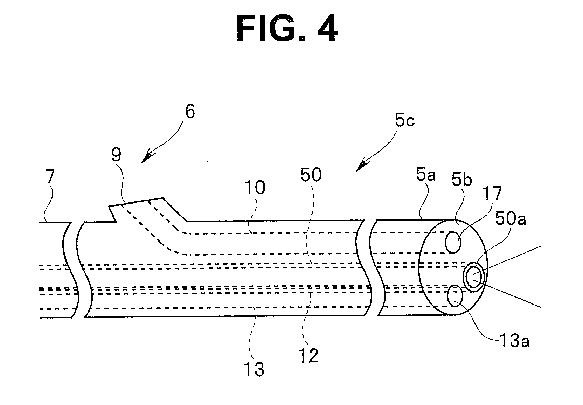

Next, a second embodiment will be described. Fig. 4 is a diagram for describing a distal end configuration of an endoscope according to the second embodiment. Note that in Fig. 4, the same components as those in Fig. 2 are assigned the same reference numerals and description thereof will be omitted.

As shown in Fig. 4, an insertion portion 5c of the endoscope 2 of the second embodiment is configured by inserting therein, a ring-shaped detection fiber 50 disposed around a circumference of the optical fiber 12 instead of the detection fiber 14 in Fig. 2. The detection fiber 50 is connected to the beam splitter 29 of the main body apparatus 3 as in the case of the detection fiber 14 of the first embodiment.

During normal observation, the detection fiber 50 receives return light from an object to be observed by a distal end face 50a and guides the received return light to the beam splitter 29. On the other hand, during auxiliary observation, the detection fiber 50 guides illuminating light emitted from the auxiliary light source 31 and radiates the illuminating light from the distal end face 50a onto the object to be observed. The rest of the configuration and operation are similar to those of the first embodiment.

With such a configuration, the endoscope system 1 of the present embodiment has effects similar to those of the first embodiment and also has an effect of being able to reduce the diameter of the insertion portion 5c compared to the insertion portion 5 of the first embodiment.

Note that the respective steps in the flowcharts of the present specification may be executed in different order of execution or a plurality of the steps may be executed simultaneously or the steps may be executed in different order every time of execution unless contrary to the nature thereof.

The present invention is not limited to the aforementioned embodiments, but can be changed or modified in various ways without departing from the spirit and scope of the present invention.

The present application claims a priority based on Japanese Patent Application No. 2013-195740, filed on September 20, 2013, the disclosure of which is incorporated in the present specification, the scope of claims and the drawings by reference in its entirety.

## Claims

1. An endoscope system comprising:
an elongated insertion portion that is inserted into a portion to be observed;
a first light-receiving section that is provided at a distal end portion of the insertion portion and can receive light from an object to be observed;
a first light transmitting section that is inserted into the insertion portion and transmits the light received by the first light-receiving section;
a first light guide section that divides the light transmitted by the first light transmitting section into a first optical path and a second optical path, and can guide the light;
a second light-receiving section that is provided at the distal end portion and can receive the light from the object to be observed;
a second light transmitting section that is inserted into the insertion portion and transmits the light received by the second light-receiving section;
a second light guide section that reflects the light transmitted by the second light transmitting section into a third optical path oriented in a same direction as the first optical path, can guide the light, and can make light from a light source provided on a proximal end side of the second light transmitting section incident on a proximal end portion of the second light transmitting section;
a photodetector that can detect light intensities of the light from the first optical path and the third optical path; and
an image pickup device that can receive the light from the second optical path.

2. The endoscope system according to claim 1, comprising:
a display apparatus that can display either a first output according to information acquired by the photodetector or a second output according to an image acquired by the image pickup device,
wherein which of the first output or the second output is to be displayed on the display apparatus is selectable.

3. The endoscope system according to claim 2, wherein when the second output is selected, light from the light source is made incident on the second light guide section.

4. The endoscope system according to claim 1, wherein the insertion portion comprises an illuminating light radiation section that can scan and emit laser light.

5. The endoscope system according to claim 4, wherein the illuminating light radiation section comprises an optical fiber that emits laser light and a drive section that can scan the optical fiber.

6. The endoscope system according to claim 4, comprising:
a detection section that can detect whether there is any abnormality in scanning of laser light in the illuminating light radiation section,
wherein the detection section drives the light source and the image pickup device based on a detected abnormality.

7. The endoscope system according to claim 4, comprising:
a starting section that drives, when there is an abnormality in scanning of laser light in the illuminating light radiation section, the light source and the image pickup device.

8. The endoscope system according to claim 7, wherein the starting section operates only when there is an abnormality in scanning of the laser light.

9. The endoscope system according to claim 6, wherein the detection section stops emission of the laser light when the abnormality is detected.
